# EUROPEAN PATENT SPECIFICATION

(11) **EP 2 809 229 B1**
(45) Date of publication and mention of the grant of the patent: **20.03.2019**
(21) Application number: 12867263.1
(22) Date of filing: 24.08.2012
(51) Int. Cl.: A61B 5/05

(54) **MRI TRANSFER STATION**
MRT-ÜBERTRAGUNGSSTATION
STATION DE TRANSFERT D'IRM

(30) Priority: 02.02.2012 US 201261593911 P
(43) Date of publication of application: 10.12.2014
(73) Proprietor: Children's Hospital Medical Center, Cincinnati, OH 45229-3039 (US)
(72) Inventor: DUMOULIN, Charles L., Cincinnati, OH 45229 (US)
(74) Representative: Williams, Michael Ian
(86) International application number: PCT/US2012/052242
(87) International publication number: WO 2013/115846

(56) References cited:
- US-A- 5 178 146
- US-A- 5 724 970
- US-A1- 2006 293 589
- US-A1- 2011 113 555
- US-B2- 6 611 702
- US-B2- 7 599 728
- US-B2- 7 850 595

## Description

### CROSS-REFERENCE TO RELATED APPLICATIONS

The present disclosure claims priority benefit of United States Provisional Patent Application Ser. No. 61/593,911 filed February 2, 2012 and is further related to United States Provisional Patent Application Ser. No. 61/429,567 filed January 4, 2011.

### FIELD OF THE DISCLOSURE

The present disclosure relates generally to magnetic resonance imaging equipment suitable for use in neonatal care, and, more particularly, to a transfer station for receiving an infant from an incubator and to assist with preparing the infant for an MR scan.

### BACKGROUND OF THE DISCLOSURE

Today premature infants weighing less than 1 kg may be stabilized and allowed to develop in neonatal intensive care units (NICUs). Magnetic resonance imaging (MRI) is a powerful technique for diagnostic purposes but is not routinely performed on these infants due to a number of technical and logistical challenges.

When a mother has a high-risk pregnancy, it is possible that the baby could be born prematurely and would require treatment in a neonatal intensive care unit or NICU. Also, unexpected early delivery may require that an infant be treated in an NICU.

One difficulty in utilizing MRI for these premature infants is monitoring the vital signs and life support of the infant during MR examination. Parameters that must be monitored during examination include electrolyte levels, hydration and temperature. A second difficulty in utilizing MRI is that the infant must be moved from an incubator or isolette into and out of the MR scanner. This movement places the infant at risk for injury.

Despite challenges, MRI has the potential to play an important diagnostic role in the care and management of neonates. The full use of this imaging technique requires that the imaging take place as early as the first few hours of life. At this stage, however, the infants are hemodynamically unstable. Accordingly, transporting and maintaining homeostasis in these fragile infants presents difficulty.

Another challenge in using MRI for neonates is that MRI systems are frequently located in Radiology departments outside of and perhaps distant from the NICU. Consequently, the neonate must be escorted out of the NICU. This may present certain logistical and technical challenges with respect to controlling the neonate's environment. Furthermore, removing staff from the NICU to transfer and attend to one baby outside the NICU can place the remaining babies in the NICU at increased risk of a reduced level of care due to decreased staff coverage.

An MR compatible transport incubator and imaging system has been developed (Dumoulin et. al.) and is currently in use. Concepts in Magnetic Resonance (Magnetic Resonance Engineering), Vol.15 (2) 117-128 (2002). This system is a self-contained MR compatible transport incubator which carries the infant from the NICU to an MR scanner located in or near the NICU. With this approach the baby must first be transferred from its "home" incubator or isolette in the NICU into the transport incubator. The transport incubator is then moved to the MR scanner where it is docked with the scanner. A portion of the transport incubator containing the baby is then moved into the center of the MR imaging system magnet where MR imaging is performed. While this approach has the advantage of not disturbing the baby while it is in the transport incubator, even during MR scanning, it has several limitations including: a) infants must be fully detached from the monitoring equipment in their home incubator to be transferred into the transport incubator, b) the MR system that is used for imaging must have a bore large enough to accommodate the portion of the transport incubator containing the baby (thereby requiring a large heavy magnet), c) the baby and its attending staff need to leave the NICU for scanning, and d) because the transport incubator must be fully MR compatible while providing full life support for the baby, the system is heavy and expensive.

An alternate approach to provide MR imaging to newborn babies has been disclosed by Feenan in U.S. Patent 7,599,728. In this approach a relatively smaller MR magnet is employed and MR-compatible incubators are docked to the magnet thereby permitting the baby to be slid into the magnet for imaging. While this approach has the benefit of providing a magnet that is more easily installed in the NICU, it does have several limitations including the need for MR compatible incubators to be used throughout the NICU, or the transfer of a neonate from a non-MR-compatible home incubator to an MR-compatible incubator. This approach also limits the access to the attending staff as they prepare the infant for MR scanning. In particular, the staff must reach through the incubator to push the baby into and out of the magnet.

In view of the foregoing, it may be understood that improved techniques for neonatal care necessitate improved transfer techniques for neonates in NICUs. In particular there is a need for an MR neonatal imaging system that can be easily sited in the NICU. The NICU magnet should be small, lightweight and acoustically quiet to permit installation within the physical boundaries of the NICU. Furthermore, there is a need for MR imaging of neonates without requiring them to be transferred out of their home incubators, or detaching them from their physiological monitoring systems or intravenous tubes. There is also a need to minimize physical movement of the baby as it enters the MR magnet and to ensure that it stays still during MR scanning. There is an additional need to for a neonatal MR imaging system that will allow babies in the NICU to be imaged without requiring that incubators in the NICU be MR compatible.

US 2006/0293589 discloses a system for transferring a patient into an imaging system. The system includes a transfer board and a cradle. A patient rests on the transfer board. The transfer board and the cradle are interconnected for movement within an imaging system, such as an MRI bore of an MRI system.

### SUMMARY

According to a first aspect of the present invention there is provided a transfer station adapted for use in association with an MR scanner and an incubator for neonatal infants as defined in claim 1.

According to a second aspect of the present invention there is provided a method of providing a neonatal infant with an MR imaging scan as defined in claim 13. Preferred features of the invention are recited in the dependent claims.

The present disclosure will now be described in more detail with reference to exemplary embodiments thereof as shown in the accompanying drawings. While the present disclosure is described below with reference to exemplary embodiments, it should be understood that the present disclosure is not limited thereto. Those of ordinary skill in the art having access to the teachings herein will recognize additional implementations, modifications, and embodiments, as well as other fields of use, which are within the scope of the present disclosure as described herein, and with respect to which the present disclosure may be of significant utility.

### BRIEF DESCRIPTION OF THE DRAWINGS

In order to facilitate a fuller understanding of the present disclosure, reference is now made to the accompanying drawings, in which like elements are referenced with like numerals. These drawings should not be construed as limiting the present disclosure, but are intended to be exemplary only.
Figure 1a is a perspective view of an exemplary MR imaging system, an exemplary MRI transfer station, and an exemplary incubator containing a neonate for use with the present disclosure;
Figure 1b is a perspective view of the present disclosure showing the removal of a portion of the incubator before preparing the neonate for movement and MR scanning;
Figure 1c is a perspective view showing the placement of the neonate on the MRI transfer station;
Figure Id is a perspective view showing the placement of the neonate into the exemplary MR imaging system;
Figure 2 shows a partially exploded perspective view of one embodiment of the MRI transfer station (with cover) suitable for preparing the neonate for MR imaging;
Figure 3 shows a top-plan view of an MR magnet and incubator with barrier poles to limit access of the incubator to the magnet; and
Figure 4 shows a top-plan view of an MR magnet and incubator with an elevated floor barrier to limit access of the incubator to the magnet.

### DETAILED DESCRIPTION

The use of MRI techniques for infants, and in particular neonates, is highly desirable. MRI techniques provide diagnostic information without patient exposure to ionizing radiation, and are suitable for extended and repeated studies.

MR techniques provide excellent anatomic visualization and functional information. They can be used to measure neural fiber track development and have a number of potential clinical uses including, but not limited, to diagnosis of brain trauma, cardiac abnormalities, congenital defects and the assessment of lung development.

There are, however, a number of challenges in the use of MRI for neonatal imaging. Patient access during scanning can be difficult as MR magnets are typically large and surround the patient. Safety concerns include forces on ferromagnetic objects, potential for rf heating and acoustic noise. Also, logistics may be difficult, as MR scanners tend to be in radiology departments, while neonate infants are typically in the NICU.

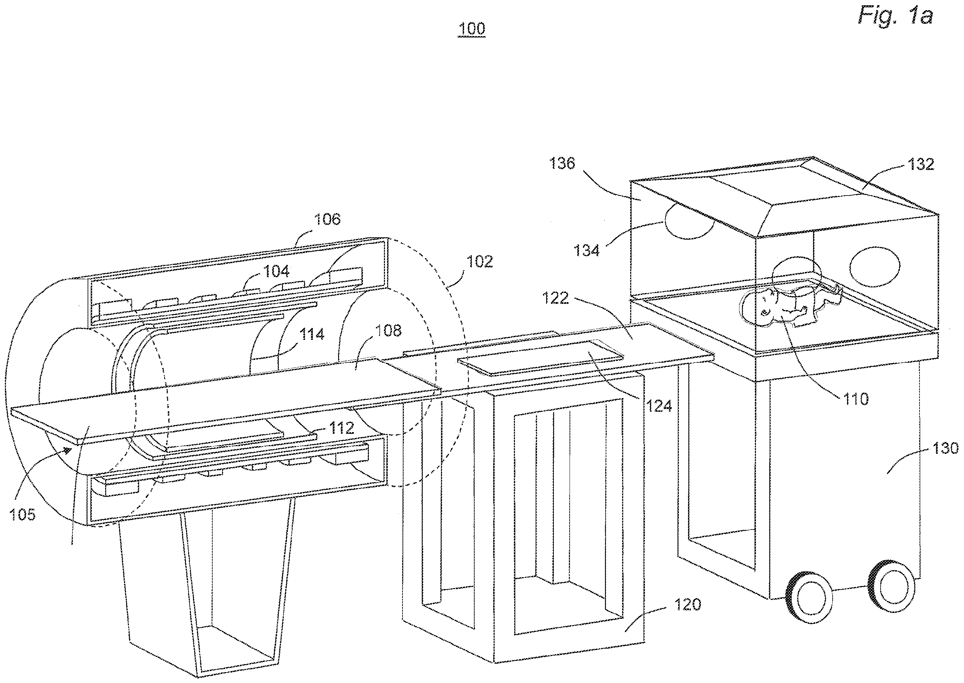

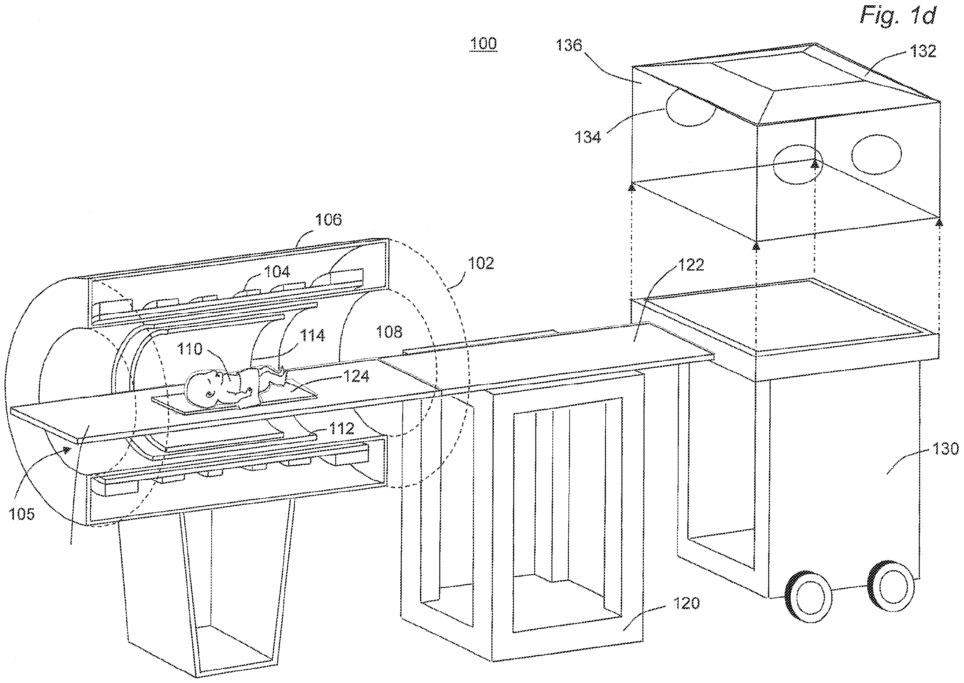

Figures 1a-1d depict an exemplary MRI and incubator system 100 in or for which the techniques for the MR imaging of neonates in accordance with the present disclosure may be implemented. The illustrated MRI system comprises an MRI scanner 102. Since the components and operation of the MRI scanner are well-known in the art, only some basic components helpful in the understanding of the system 100 and its operation will be described herein.

The MRI scanner 102 may comprise a cylindrical superconducting magnet 104, which generates a static magnetic field within a bore 105 of the superconducting magnet 104. The superconducting magnet 104 generates a substantially homogeneous magnetic field within the magnet bore 105. The superconducting magnet 104 may be enclosed in a magnet housing 106.

A set of cylindrical magnetic field gradient coils 112 may also be provided within the magnet bore 105. The gradient coils 112 can generate magnetic field gradients of predetermined magnitudes, at predetermined times, and in three mutually orthogonal directions within the magnet bore 105. With the field gradients, different spatial locations can be associated with different precession frequencies, thereby giving an MR image its spatial resolution. An RF transmitter coil 114 is positioned within the gradient coils 112. The RF transmitter coil 114 emits RF energy in the form of a magnetic field for the purpose of exciting MR signals during image acquisition. The RF transmitter coil 114 can also receive MR response signals. The MR response signals are amplified, conditioned and digitized into raw data as is known by those of ordinary skill in the art.

The present disclosure provides an apparatus and a technique for safely and effectively transferring an infant from the primary care area, such as an incubator, to the MR magnet. In one particular embodiment, the present disclosure provides the means to transfer a neonate from the NICU to an MR magnet located either in a radiology department or in the NICU itself. The present disclosure may accomplish this by providing an MR-compatible transfer station that may be permanently or releasably attached to the MR magnet. This station may create an MR compatible environment that, if desired, the baby may be moved into without being detached from patient monitoring or life support systems. Once stabilized on the transfer station, the baby may then be moved into the magnet for imaging. Note that in the present disclosure the incubator does not need to be fully MR compatible and may be constructed with some MR incompatible elements such as electrical motors.

Referring again to Figures 1a-1d, an incubator 130 containing a neonate 110 is shown. The incubator 130 may have a top 132 that can be left in place or alternatively moved away from the neonate. The walls of the incubator may be clear and may include one or more incubator access ports 134 to permit attending medical staff to reach into the incubator. The incubator may have a front panel 136 that may be removed or rotated out of the way to provide wider access to the neonate 110.

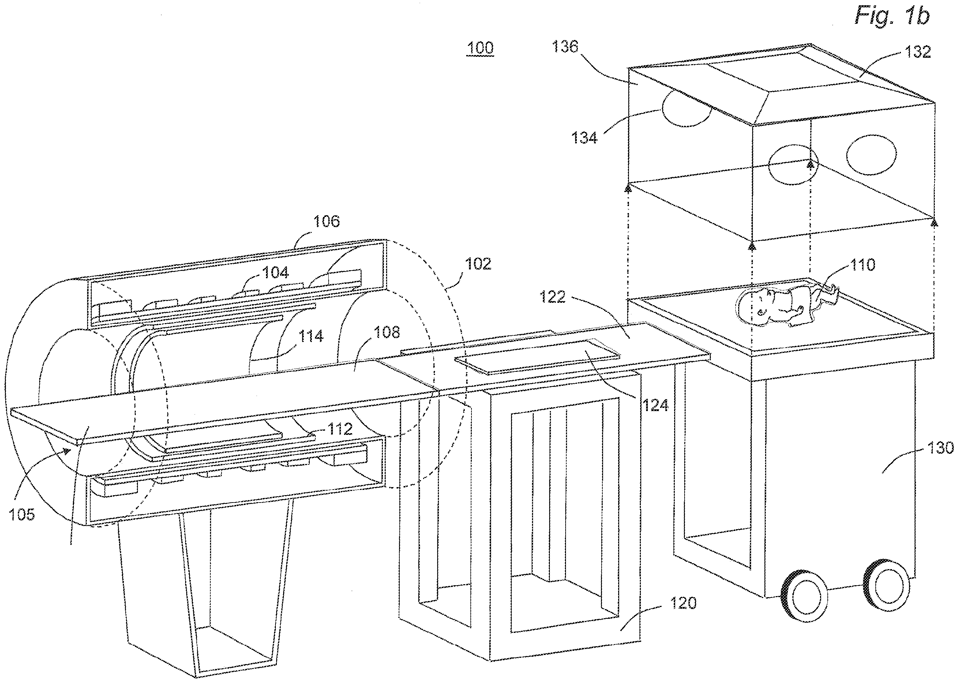

Figure 1b shows the elevation and removal of the top portion of the incubator 130 so as to provide access to the neonate. Alternate means to gain access to the neonate may include folding down or removing one or more side panels, or front panel 136, of the incubator 130.

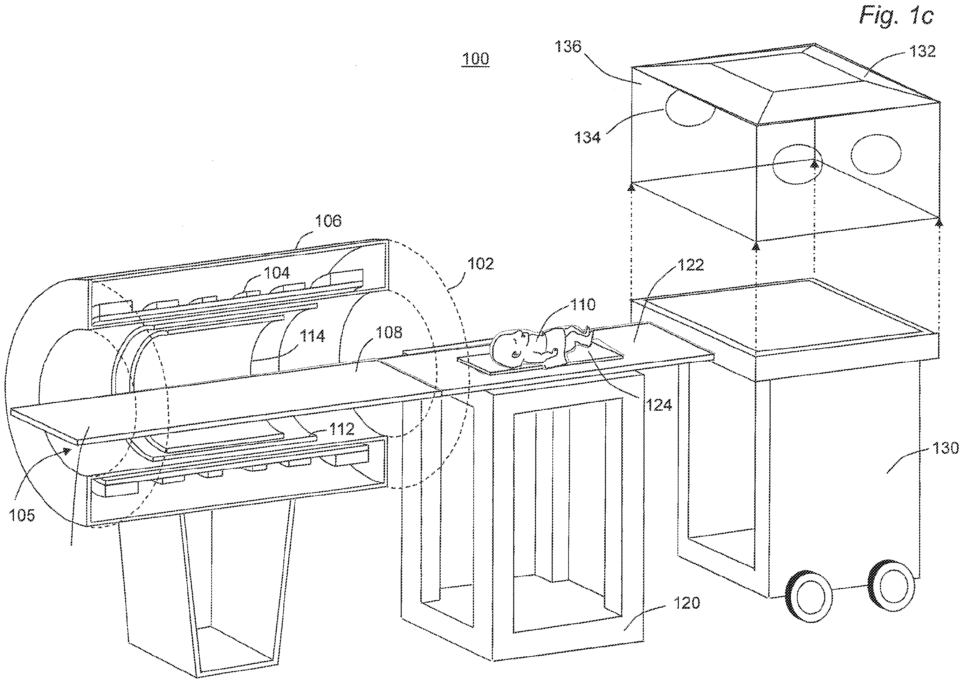

Figure 1c shows the placement of the neonate 110 onto a transfer station bed 124 on a transfer station surface 122. The transfer station surface 122 may be supported on a transfer station base 120. In the current embodiment of the present disclosure, neonate 110 may be prepared for MR scanning while in this location. Preparations may include safety checks, swaddling, attachment of additional monitoring equipment, placement of MR receive coils, and/or attachment of hearing protection to the neonate 110. In one embodiment of the disclosure the incubator 130 may be docked or mechanically attached to the transfer station. In another embodiment the incubator 130 may be placed near the transfer station, but does not physically contact it.

Figure Id shows the insertion of the neonate 110 and transfer station bed 124 into the MR imaging system 102. Note that during insertion the transfer station bed 124 moves off of transfer station surface 122 and onto a magnet bed support surface 108. Note also that during this insertion the neonate is not moved with respect to the transfer station bed 124 although the transfer station bed is moved into the MR imaging system with the neonate.

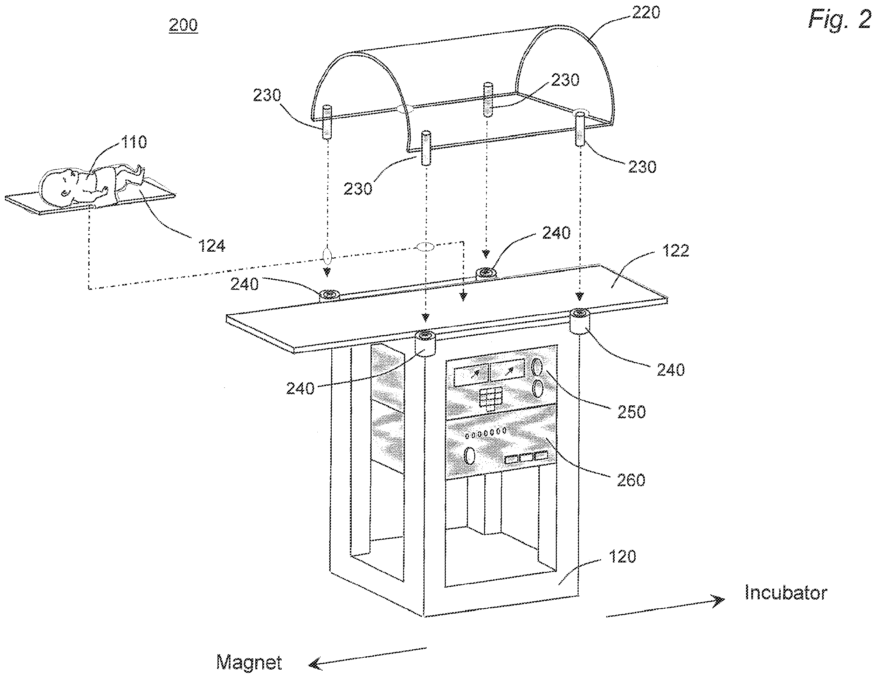

Figure 2 shows one embodiment of a transfer station 200 in greater detail. The transfer station 200 may include the transfer station surface 122, a transfer station base 120 and a transfer station cover 220. The transfer station base 120 and the transfer station surface 122 may be releasably attached to the MRI scanner 102, or if desired be movable with an option to latch into a selected position. The transfer station cover 220 may incorporate one or more cover locking pins 230 that are adapted for insertion into a like number of corresponding cover pin sockets 240. The cover locking mechanism shown in the figures is exemplary. It should be understood that alternate mechanisms for attaching the transfer station cover 220 to the transfer station base 120, including hinges, slots, clamps and slides are covered by the spirit of the disclosure. If desired, neonate access ports similar to the incubator access ports 134 found in the incubator 130 may be incorporated into the transfer station cover 220. It should be obvious to one skilled in the art that the transfer station may be constructed using MR compatible materials.

The present disclosure is particularly advantageous in that it minimizes the transfer time from the NICU to MR imaging system 102 and may provide less stress on the infant. Another advantage of the present disclosure is that babies may not need to be fully detached from their home incubator, which is shown as incubator 130 in Figures 1a-1d. Physiological monitoring leads, IV tubes, ventilator tubes and temperature sensors may be left in place. This may further reduce preparation times and stress on the infant. Once the baby is stabilized, the baby and the transfer table bed 124 may be inserted into the magnet to place the neonate 110 in the imaging region of the MRI scanner. This may be done manually or with an appropriate drive system in which a motor moves the transfer table bed 124. This approach ensures that the local environment of the neonate 110 is not altered as it is brought into the center of the imaging system. Furthermore, because a transport incubator is not required, and since the home incubator need not be fully MR-compatible, the MR magnet may be relatively smaller and lightweight. This may make it more easily installed within the confines of the NICU, and may provide improved access to MR scanning for premature babies. In addition, the present disclosure may permit MR scanning to be performed with fewer support personnel, and/or places support personnel closer to the other babies present in the NICU. With the present disclosure MR imaging may be made available to all babies in a NICU (typically between 10 and 60) using a single MR magnet and a single transfer station that may be used with each compatible incubator in the NICU.

In particular, the present disclosure relates to a transfer station for preparing an infant, including neonates, prior to transfer into the magnet. In the present disclosure the transfer station may be an auxiliary incubator that is both MR-compatible and if desired, permanently or releasably attached to the MRI scanner 102. The transfer station may have all of the functionality of the neonate's home incubator, but implemented in an MR-compatible and MR-safe manner.

Once the infant is on the transfer station 200, the infant can be prepared for MR scanning. As will be recognized by those skilled in the art of MR scanning, MR scanning frequently requires that several steps be performed before a patient may be inserted into an imaging magnet. These steps may include: a) immobilization of the patient (in the case of neonatal imaging, swaddling is frequently sufficient), b) the optional insertion of IV tubes for contrast injections, c) the attachment of MR imaging coils, d) a safety check to verify that no ferromagnetic objects are present, e) placement of hearing protection, and f) verification of patient stability and comfort. All of these steps may be performed while the patient is near the magnet, and may require access to the patient which is not always possible with most incubator designs.

Another aspect of the transfer station 200 of the present disclosure is that it may provide full environmental control for the neonate. Many neonates are too young to be able to fully control their internal temperature, and it is well known to those skilled in the art that small neonates must be kept warm. In the present disclosure, this may be done with warm air and/or a radiant heater driven by a temperature controller 250. In one embodiment of the disclosure, a thermocouple or similar temperature sensor modified for use in the MR environment with non-ferromagnetic parts and appropriate rf filtering may be used to provide feedback to the temperature controller 250 to provide suitable temperature control. In another embodiment of the present disclosure a physiologic monitoring system 260 may be provided. This system may be MR compatible and MR safe. It can be used if desired in place of the patient monitoring systems found in the neonate's home incubator.

The transfer station 200 of the present disclosure may be placed between incubator 130 and MRI system 102. One major function of the transfer station may be to act as a buffer element to prevent non MR-compatible objects from entering into the magnet while the baby is inside the magnet.

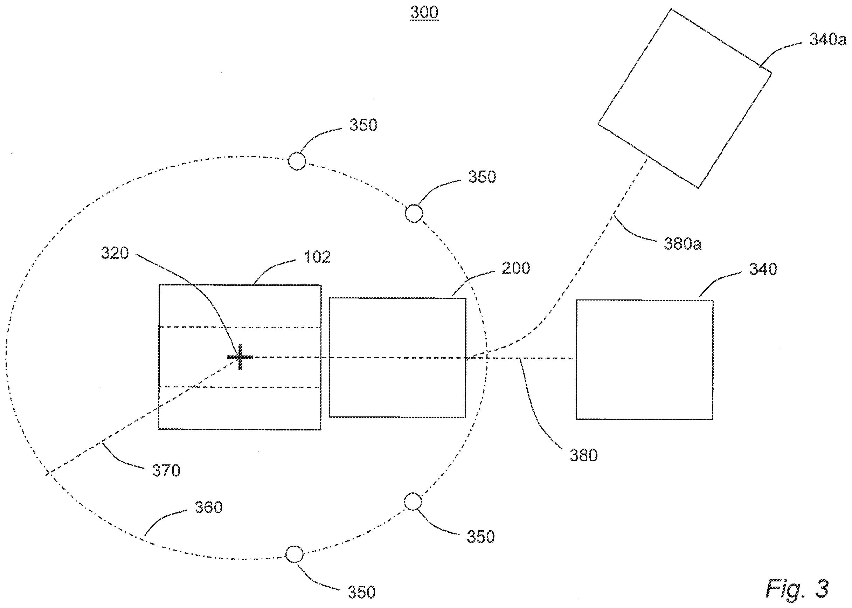

As shown in Figure 3, the safety barrier provided by the transfer station 200 can be augmented with in-room barriers such as poles 350 in the floor spaced to prevent incubators or other devices from getting within a predetermined fringe magnetic field strength 360 (typically chosen to be 5 Gauss) at a selected distance 370 of the magnet. With such an approach, the incubator 130 may be placed into a safe position 340 from which the neonate 110 is moved and prepared for MR scanning in the transfer station 200. With the incubator in safe position 340 the neonate 110 is moved towards MR scanner 102 along an axial route 380. Alternatively, the incubator can be placed in an alternate safe position 340a and the neonate 110 moved to the transfer station 200 along alternate route 380a. Once the neonate 110 is prepared for MR scanning, it can be moved to the isocenter 320 of the MR scanner 102.

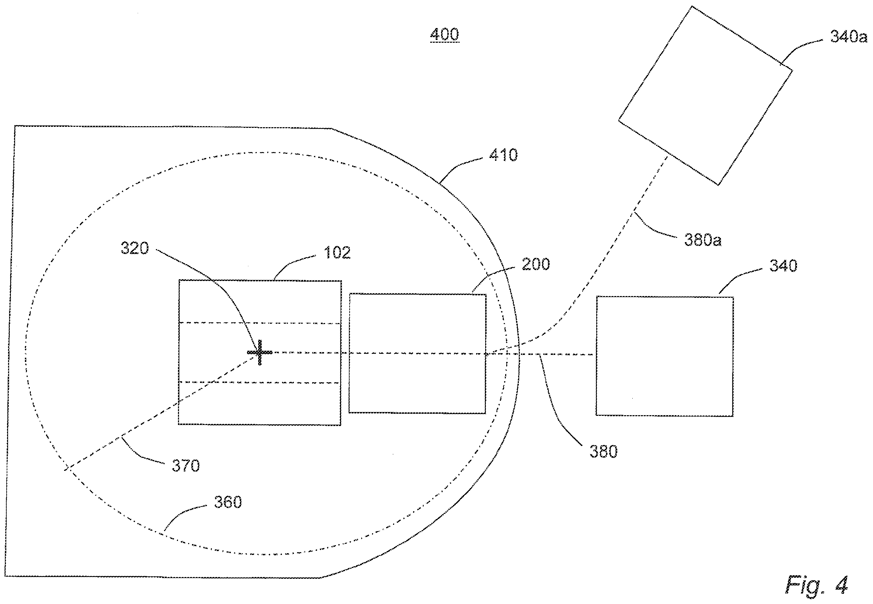

Alternatively, the barrier can be augmented with a step design as shown in Figure 4 in which the floor that accommodates the NICU incubator is lower than a raised floor 410 surrounding the magnet. The step created by the two levels of the floor acts to prevent the incubator from approaching too closely to the magnet.

The present disclosure has the advantage of working with incubators and isolettes that do not require modification to be made MR compatible. According to the present disclosure, the infant may, in one embodiment, still be tethered to the incubator by life support and monitoring methods, for example IV lines and EKG leads. Alternatively, the transfer station 200 itself may be equipped with the aforementioned and other life support and monitoring methods.

The transfer station may in one embodiment, as shown in Figure 1b and Figure 1c, be open like an isolette. In one embodiment, the open isolette may be configured with radiant heating. In another embodiment, the transfer station may be enclosed like an incubator with the addition of a cover. In one embodiment, the enclosed transfer station may be configured to provide warm circulating air.

In one embodiment of the disclosure, the incubator may be adapted to dock to the transfer station. This may be accomplished for instance by a mechanical latch which rigidly engages and requires a physical action to unlock, or it could be a simple "tongue and groove" arrangement in which the incubator can be brought close to the magnet with a selected alignment. In such an arrangement, it may be desirable to engage the wheel brakes on the incubator during docking to prevent the incubator from moving unexpectedly.

As mentioned, in one embodiment, the transfer station 200 may be equipped to include life support and monitoring equipment. Such equipment includes, but is not limited to, EKG monitoring, IV tubes, oxygen monitors, ventilators, breathing gases, and bilirubin treatment. If needed, the transfer station 200 can be powered by an external supply or an on-board MR-compatible battery.

In one embodiment of the disclosure, the transfer station 200 may include physical barriers to prevent extraneous objects being sucked into the infant when the infant is inside the magnet. In one embodiment, the barrier may include a full enclosure made of clear engineering plastic that is resistant to impact damage. This feature may provide full visual access of the baby but may provide a barrier to the entry of other objects into the magnet.

In one embodiment, a Faraday cage may be built into the transfer station 200 to prevent RF interference from degrading the MR image. This would be particularly advantageous if the MR system is not placed in an RF screen room. Should a Faraday cage be incorporated into the transfer station, an internal rf tight panel or door may need to be added between the magnet and the transfer station. It may also be desirable to provide penetration filters for monitoring leads to minimize rf interference during MR imaging. In an even further embodiment, the transfer station may incorporate a scale for weighing the infants.

While the foregoing description includes many details and specificities, it is to be understood that these have been included for purposes of explanation only, and are not to be interpreted as limitations of the present disclosure. It will be apparent to those skilled in the art that other modifications to the embodiments described above may be made without departing from the scope of the disclosure. Accordingly, such modifications are considered within the scope of the disclosure as intended to be encompassed by the following claims.

## Claims

1. A transfer station adapted for use in association with an MR scanner and an incubator for neonatal infants, said transfer station comprising:
a transfer station base (120);
a transfer station surface (122) supported on said transfer station base (120) wherein said transfer station surface (122) is disposed at substantially the same height as the height of a corresponding patient surface in said incubator (130);
a transfer station bed (124) movably positioned atop said transfer station surface (122);
a releasable attachment mechanism configured to align and releasably attach said transfer station (200) to at least one of an incubator (130) and an MR scanner (102);
a transfer station cover (220) adapted to be releasably attached to said transfer station base (120); and
a drive system adapted to move said transfer station bed (124) into and out of said MR scanner (102).

2. The transfer station as defined in claim 1, wherein said transfer station cover (220) is attached by one or more hinges to said transfer station base (120).

3. The transfer station as defined in claim 1, wherein said transfer station cover (220) is releasably attached to said transfer station base (120) by one or more locking pins (230).

4. The transfer station as defined in any of the preceding claims, wherein said transfer station cover (220) defines at least a partially enclosed patient space when attached to said transfer station base (120).

5. The transfer station of any of the preceding claims, wherein the transfer station (200) is equipped with life support and monitoring capabilities, and/or wherein the transfer table is configured as an open isolette, and/or wherein the transfer station further comprises at least one of a scale for weighing the neonatal infant and a Faraday cage.

6. The transfer station as defined in any of the preceding claims, wherein said releasable attachment mechanism comprises at least one of a tongue and groove arrangement and mechanical latch.

7. A system for providing neonatal infants with magnetic resonance image testing, the system comprising:
a) an incubator (130) for neonatal infants;
b) an MR scanner (102); and
c) a transfer station (200) according to any of the preceding claims.

8. The system of claim 7, wherein said incubator (130) is at least partially enclosed and comprises at least one of a removable cover (132) and at least one removable wall (136), and wherein said attachment mechanism is further configured to releasably attach said transfer station (200) to said incubator (130) adjacently with a side of the incubator (130).

9. The system of claim 7 or 8 further comprising at least one safety barrier adapted to prevent non-MRI compatible objects from entering into the MR scanner (120).

10. The system of claim 9 wherein said safety barrier comprises in-room barriers disposed on the floor about the system to prevent incubators (130) and other devices from getting within a predetermined distance from the MR scanner (102) and/or wherein said safety barrier comprises at least one of a series of poles (350) disposed on the floor about the MR scanner (102) and a wall disposed on the floor about the MR scanner (102).

11. The system of claim 9 or 10, wherein said safety barrier is spaced at a selected distance (370) from the MR scanner (102) that corresponds to a predetermined fringe magnetic field strength (360) of the MR scanner (102).

12. The system of any of claims 9 to 11, wherein said safety barrier comprises a step design in which the floor that accommodates the incubator (130) is lower than a raised floor (410) surrounding the MR scanner (102).

13. A method of providing a neonatal infant with an MR imaging scan, the method comprising the steps of:
a) providing an incubator (130) with a neonatal infant (110) therein, said incubator (130) being at least partially enclosed and further comprising at least one of a removable cover (132) and a removable wall (136);
b) removing the removable cover (132) or removable wall (136);
c) attaching a transfer station (200) next to said incubator (130) adjacent to an open side of the incubator (130), said transfer station (200) being adapted to releasably attach to said incubator (130) and to support a neonatal infant (110) thereon, said transfer station comprising a transfer station base (120) and a transfer station surface (122) attached to a top surface of said transfer station base (120) wherein said transfer station surface (122) is disposed at substantially the same height as the height of a corresponding patient surface in said incubator (130); said transfer station further comprising a transfer station bed (124) moveably positioned atop said transfer station surface (122); said transfer station further comprising a releasable attachment mechanism to align and releasably attach said transfer station (200) to the incubator (130), and a drive system adapted to move said transfer station bed (124) into and out of said MR scanner (102);
d) transferring the neonate infant (110) from the incubator (130) onto the transfer station bed (124);
e) positioning said transfer station (200) adjacent to an MR scanner (102);
f) preparing the neonate infant (110) for MR scanning;
g) positioning said neonate infant (110) within said MR scanner (102) by operating said drive system of the transfer station to move said transfer station bed (124) and said neonate infanct (110) into the chamber of the MR scanner (102); and
h) activating the MR scanner (102) to obtain MR imaging of the neonate infant (110).

14. The method of claim 13, wherein said step of preparing the neonate infant (110) for MR scanning includes at least one of:
a) immobilizing the neonate infant (110);
b) providing the neonate infant (110) with hearing protection;
c) executing a safety check of the MR scanner (102) and the neonate infant (110) to verify that no extraneous ferromagnetic objects are present; and
d) verifying the neonate infant's (110) stability.

## Patentansprüche

1. Umlagerungsstation, die für die Verwendung im Zusammenhang mit einem MR-Scanner und einem Inkubator für neugeborene Säuglinge angepasst ist, wobei die Umlagerungsstation Folgendes umfasst:
eine Umlagerungsstationsbasis (120);
eine Umlagerungsstationsoberfläche (122), die auf der Umlagerungsstationsbasis (120) gelagert ist, wobei die Umlagerungsstationsoberfläche (122) im Wesentlichen auf derselben Höhe wie die Höhe einer entsprechenden Patientenoberfläche in dem Inkubator (130) angeordnet ist;
ein Umlagerungsstationsbett (124), das bewegbar auf der Umlagerungsstationsoberfläche (122) positioniert ist;
einen lösbaren Befestigungsmechanismus, der konfiguriert ist, um die Umlagerungsstation (200) an einem Inkubator (130) und/oder an einem MR-Scanner (102) auszurichten oder sie daran lösbar zu befestigen;
eine Umlagerungsstationsabdeckung (220), die angepasst ist, um lösbar an der Umlagerungsstationsbasis (120) befestigt zu werden; und
ein Antriebssystem, das angepasst ist, um das Umlagerungsstationsbett (124) in den und aus dem MR-Scanner (102) zu bewegen.

2. Umlagerungsstation nach Anspruch 1, wobei die Umlagerungsstationsabdeckung (220) durch ein oder durch mehrere Scharniere an der Umlagerungsstationsbasis (120) befestigt ist.

3. Umlagerungsstation nach Anspruch 1, wobei die Umlagerungsstationsabdeckung (220) durch einen oder durch mehrere Sperrstifte (230) an der Umlagerungsstationsbasis (120) lösbar befestigt ist.

4. Umlagerungsstation nach einem der vorhergehenden Ansprüche, wobei die Umlagerungsstationsabdeckung (220) einen wenigstens teilweise eingeschlossenen Patientenraum definiert, wenn sie an der Umlagerungsstationsbasis (120) befestigt ist.

5. Umlagerungsstation nach einem der vorhergehenden Ansprüche, wobei die Umlagerungsstation (200) mit Lebenserhaltungs- und -überwachungsfähigkeiten ausgestattet ist, und/oder wobei der Umlagerungstisch als eine offene Isolette konfiguriert ist, und/oder wobei die Umlagerungsstation ferner eine Waage zum Wiegen des neugeborenen Säuglings und/oder einen Faraday-Käfig umfasst.

6. Umlagerungsstation nach einem der vorhergehenden Ansprüche, wobei der lösbare Befestigungsmechanismus eine Nut-und-Feder-Anordnung und/oder eine mechanische Feststelleinrichtung umfasst.

7. System zum Versorgen von neugeborenen Säuglingen mit magnetischer Resonanzbilduntersuchung, wobei das System Folgendes umfasst:
a) einen Inkubator (130) für neugeborene Säuglinge;
b) einen MR-Scanner (102); und
c) eine Umlagerungsstation (200) nach einem der vorhergehenden Ansprüche.

8. System nach Anspruch 7, wobei der Inkubator (130) wenigstens teilweise eingeschlossen ist und wenigstens eine entfernbare Abdeckung (132) und wenigstens eine entfernbare Wand (136) umfasst, und wobei der Befestigungsmechanismus ferner konfiguriert ist, um die Umlagerungsstation (200) an den Inkubator (130) angrenzend an einer Seite des Inkubators (130) lösbar zu befestigen.

9. System nach Anspruch 7 oder 8, umfassend wenigstens eine Sicherheitsabsperrung, die angepasst ist, um zu verhindern, dass nicht mit MRI kompatible Objekte in den MR-Scanner (120) eintreten.

10. System nach Anspruch 9, wobei die Sicherheitsabsperrung innen gelegene Barrieren umfasst, die auf dem Boden um das System herum angeordnet sind, um zu verhindern, dass Inkubatoren (130) und andere Vorrichtungen innerhalb eine vorbestimmte Entfernung von dem MR-Scanner (102) gelangen, und/oder wobei die Sicherheitsabsperrung eine Reihe von Stangen (350), die auf dem Boden um den MR-Scanner (102) herum angeordnet sind, und/oder eine Wand umfasst, die auf dem Boden um den MR-Scanner (102) herum angeordnet ist.

11. System nach Anspruch 9 oder 10, wobei die Sicherheitsabsperrung in einer ausgewählten Entfernung (370) von dem MR-Scanner (102) beabstandet ist, die einer vorbestimmten Randmagnetfeldstärke (360) des MR-Scanners (102) entspricht.

12. System nach einem der Ansprüche 9 bis 11, wobei die Sicherheitsabsperrung einen Stufenaufbau umfasst, in dem der Boden, der den Inkubator (130) aufnimmt, niedriger als ein erhöhter Boden (410) ist, der den MR-Scanner (102) umgibt.

13. Verfahren zum Versorgen eines neugeborenen Säuglings mit einem MR-Abbildungsscan, wobei das Verfahren die folgenden Schritte umfasst:
a) Versehen eines Inkubators (130) mit einem neugeborenen Säugling (110) darin, wobei der Inkubator (130) wenigstens teilweise eingeschlossen ist und ferner eine entfernbare Abdeckung (132) und/oder eine entfernbare Wand (136) umfasst;
b) Entfernen der entfernbaren Abdeckung (132) oder der entfernbaren Wand (136);
c) Befestigen einer Umlagerungsstation (200) neben dem Inkubator (130), angrenzend an eine offene Seite des Inkubators (130), wobei die Umlagerungsstation (200) angepasst ist, um sich an den Inkubator (130) lösbar zu befestigen und einen neugeborenen Säugling (110) darauf zu lagern, wobei die Umlagerungsstation eine Umlagerungsstationsbasis (120) und eine Umlagerungsstationsoberfläche (122) umfasst, die an einer oberen Oberfläche der Umlagerungsstationsbasis (120) befestigt ist, wobei die Umlagerungsstationsoberfläche (122) im Wesentlichen auf derselben Höhe wie die Höhe einer entsprechenden Patientenoberfläche in dem Inkubator (130) angeordnet ist; wobei die Umlagerungsstation ferner ein Umlagerungsstationsbett (124) umfasst, das bewegbar auf der Umlagerungsstationsoberfläche (122) positioniert ist; wobei die Umlagerungsstation ferner einen lösbaren Befestigungsmechanismus, um die Umlagerungsstation (200) an dem Inkubator (130) auszurichten und diese lösbar daran zu befestigen, und ein Antriebssystem umfasst, das angepasst ist, um das Umlagerungsstationsbett (124) in den und aus dem MR-Scanner (102) zu bewegen;
d) Umlagern des neugeborenen Säuglings (110) von dem Inkubator (130) auf das Umlagerungsstationsbett (124);
e) Positionieren der Umlagerungsstation (200) angrenzend an einen MR-Scanner (102);
f) Vorbereiten des neugeborenen Säuglings (110) zum MR-Scannen;
g) Positionieren des neugeborenen Säuglings (110) innerhalb des MR-Scanners (102) durch Betreiben des Antriebssystems der Umlagerungsstation, um das Umlagerungsstationsbett (124) und den neugeborenen Säugling (110) in die Kammer des MR-Scanners (102) hinein zu bewegen; und
h) Betätigen des MR-Scanners (102), um eine MR-Abbildung des neugeborenen Säuglings (110) zu erhalten.

14. Verfahren nach Anspruch 13, wobei der Schritt des Vorbereitens des neugeborenen Säuglings (110) zum MR-Scannen Folgendes enthält:
a) Immobilisieren des neugeborenen Säuglings (110);
b) Versorgen eines neugeborenen Säuglings mit Hörschutz (110);
c) Durchführen einer Sicherheitsprüfung des MR-Scanners (102) und des neugeborenen Säuglings (110), um sicherzustellen, dass keine fremden ferromagnetischen Objekte vorhanden sind; und/oder
d) Sicherstellen der Stabilität des neugeborenen Säuglings (110).

## Revendications

1. Station de transfert adaptée pour être utilisée avec un scanner RM et un incubateur pour enfants nouveau-nés, ladite station de transfert comprenant :
une base de station de transfert (120) ;
une surface de station de transfert (122) supportée par ladite base de station de transfert (120), ladite surface de station de transfert (122) étant disposée à substantiellement la même hauteur qu'une surface de patient correspondante dans ledit incubateur (130) ;
un lit de station de transfert (124) positionné de façon mobile au sommet de ladite surface de station de transfert (122) ;
un mécanisme d'attache amovible conçu pour aligner et attacher de façon détachable ladite station de transfert (200) à au moins un incubateur (130) et un scanner RM (102) ;
un couvercle de station de transfert (220) adapté pour être attaché de façon détachable à ladite base de station de transfert (120) ; et
un système de guidage conçu pour faire entrer et sortir ledit lit de station de transfert (124) dans et hors dudit scanner RM (102).

2. Station de transfert selon la revendication 1, dans laquelle ledit couvercle de station de transfert (220) est attaché par au moins une charnière à ladite base de station de transfert (120).

3. Station de transfert selon la revendication 1, dans laquelle ledit couvercle de station de transfert (220) est attaché de façon détachable à ladite base de station de transfert (120) par au moins une goupille de verrouillage (230).

4. Station de transfert selon la revendication 1, dans laquelle ledit couvercle de station de transfert (220) définit au moins un espace pour patient partiellement fermé quand il est attaché à ladite base de station de transfert (120).

5. Station de transfert selon l'une quelconque des revendications précédentes, la station de transfert (200) étant équipée de moyens d'assistance et de surveillance des constantes vitales et/ou la table de transfert étant conçue comme un incubateur chauffé ouvert et/ou la station de transfert comprenant en outre au moins un élément entre une balance pour nouveau-nés et une cage de Faraday.

6. Station de transfert selon l'une quelconque des revendications précédentes, dans laquelle ledit mécanisme d'attache amovible comprend au moins un élément entre un assemblage à rainure et languette et un loquet mécanique.

7. Système destiné à permettre un test d'imagerie par résonance magnétique sur des nouveau-nés, le système comprenant :
a) un incubateur (130) pour nouveau-nés ;
b) un scanner RM (102) ; et
c) une station de transfert (200) selon l'une quelconque des revendications précédentes.

8. Système selon la revendication 7, dans lequel ledit incubateur (130) est au moins partiellement fermé et comprend au moins un élément entre un couvercle amovible (132) et au moins une paroi amovible (136), et dans lequel ledit mécanisme d'attache est, en outre, conçu pour attacher de façon amovible ladite station de transfert (200) audit incubateur (130) de façon adjacente à un côté de l'incubateur (130).

9. Système selon la revendication 7 ou 8, comprenant en outre au moins une barrière de sécurité conçue pour empêcher tout objet incompatible avec l'IRM d'entrer dans le scanner RM (120).

10. Système selon la revendication 9, dans lequel ladite barrière de sécurité comprend des barrières intérieures disposées sur le sol autour du système pour empêcher les incubateurs (130) et autres appareils d'accéder en deçà d'une distance prédéterminée du scanner RM (102) et/ou dans lequel ladite barrière de sécurité comprend au moins un élément entre une série de poteaux (350) disposés sur le sol autour du scanner RM (102) et une paroi disposée sur le sol autour du scanner RM (102).

11. Système selon la revendication 9 ou 10, dans lequel ladite barrière de sécurité est espacée d'une distance sélectionnée (370) du scanner RM (102) qui correspond à une intensité limite de champ magnétique (360) du scanner RM (102).

12. Système selon l'une quelconque des revendications 9 à 11, dans lequel ladite barrière de sécurité comprend une conception comportant une marche, dans laquelle le sol qui accueille l'incubateur (130) est plus bas qu'un sol surélevé (410) qui entoure le scanner RM (102).

13. Procédé pour fournir un scan par imagerie RM à un nouveau-né, le procédé comprenant les étapes consistant à :
a) fournir un incubateur (130) contenant un nouveau-né (110), ledit incubateur (130) étant au moins partiellement fermé et comprenant en outre au moins un élément entre un couvercle amovible (132) et une paroi amovible (136) ;
b) enlever le couvercle amovible (132) ou une paroi amovible (136) ;
c) attacher une station de transfert (200) à côté dudit incubateur (130) adjacente à un côté ouvert de l'incubateur (130), ladite station de transfert (200) étant adaptée pour s'attacher de façon détachable audit incubateur (130) et pour supporter un nouveau-né (110) dessus, ladite station de transfert comprenant une base de station de transfert (120) et une surface de station de transfert (122) attachée à une surface supérieure de ladite base de station de transfert (120), ladite surface de station de transfert (122) étant disposée substantiellement à la même hauteur que la hauteur d'une surface de patient correspondante dans ledit incubateur (130) ; ladite station de transfert comprenant en outre un lit de station de transfert (124) positionné de façon mobile au sommet de ladite surface de station de transfert (122) ; ladite station de transfert comprenant en outre un mécanisme d'attache amovible conçu pour aligner et attacher de façon détachable ladite station de transfert (200) à l'incubateur (130), et un système de guidage conçu pour faire entrer et sortir ledit lit de station de transfert (124) dans et hors dudit scanner RM (102) ;
d) transférer le nouveau-né (110) de l'incubateur (130) sur le lit de station de transfert (124) ;
e) positionner ladite station de transfert (200) à côté d'un scanner RM (102) ;
f) préparer le nouveau-né (110) pour un balayage RM ;
g) positionner ledit nouveau-né (110) à l'intérieur du scanner RM (102) en actionnant ledit système de guidage de la station de transfert pour déplacer ledit lit de station de transfert (124) et ledit nouveau-né (110) dans la chambre du scanner RM (102) ;
h) activer le scanner RM (102) pour obtenir une imagerie RM du nouveau-né (110).

14. Procédé selon la revendication 13, dans lequel l'étape de préparation du nouveau-né (110) pour le scanner RM inclut au moins une étape parmi :
a) l'immobilisation du nouveau-né (110) ;
b) la fourniture d'une protection auditive au nouveau-né (110) ;
c) la réalisation d'un contrôle de sécurité du scanner RM (102) et du nouveau-né (110) pour vérifier qu'aucun corps étranger ferromagnétique n'est présent ; et
d) la vérification de la stabilité du nouveau-né (110).
